# EUROPEAN PATENT APPLICATION

(11) **EP 2 058 026 A1**
(43) Date of publication of application: **13.05.2009**
(21) Application number: 07745284.5
(22) Date of filing: 14.06.2007
(51) Int. Cl.: A61N 1/30

(54) **IONTOPHORESIS DEVICE**

(30) Priority: 30.08.2006 JP 2006233662
(71) Applicant: TTI ELLEBEAU, INC., Tokyo 140-0002 (JP)
(72) Inventor: YAMAUCHI, Mitsugu, Higashi-Shinagawa, Shinagawa-ku Tokyo 1400002 (JP); YAMAMOTO, Rie, Higashi-Shinagawa, Shinagawa-ku Tokyo 1400002 (JP)
(74) Representative: Twelmeier Mommer & Partner
(86) International application number: PCT/JP2007/062027
(87) International publication number: WO 2008/026364

(57) **Abstract**

An iontophoresis device (10) includes a working electrode assembly (20) and a non-working electrode assembly (40), and an organism contact surface (20A) at a tip of the working electrode assembly (20) and an organism contact surface (40A) at a tip of the non-working electrode assembly (40) are covered with a release liner (60) which may be peeled off when it is pulled in one direction along them. On a front surface side of the release liner (60), a base film (62) coupled therewith and having a size large enough to cover the front regions of the organism contact surfaces (20A, 40A), and a paste layer (64) attached to the front surface of the base film (62) are provided. When the paste layer (64) is peeled from the skin (S) by pulling the base film (62) through the release liner (60), a part of the stratum corneum or sebum in the contact region (S₁) of the skin (S) may be peeled.

## Description

### Technical Field

The present invention relates to an iontophoresis device for administering a drug ion to an organism by applying a voltage.

### Background Art

As such an iontophoresis device described above, as described in WO 0303742 publication, there is given one including a DC power supply, a working side electrode assembly, and a non-working side electrode assembly, for administering a drug solution to skin or mucosa from an ion exchange membrane of the working side electrode assembly.

The working side electrode assembly in the iontophoresis device described in the WO 0303742 publication is formed by stacking a first ion exchange membrane constituting an organism contact surface with the skin or mucosa, a drug solution holding portion, a second ion exchange membrane, a first electrolyte solution holding portion, and a working side electrode assembly in the stated order.

Besides, the non-working side electrode assembly is formed by stacking a fourth ion exchange membrane which being an organism contact surface with the skin or mucosa, a third electrolyte solution holding portion, a third ion exchange membrane, a second electrolyte solution holding portion, and a non-working side electrode in the stated order.

It is naturally required for the organism contact portions which being tips of the working side electrode assembly and the non-working side electrode assembly to effectively administer the drug solution in contact with the skin or mucosa. However, on the working side electrode assembly side, administration efficiency of the drug solution ion is largely varied depending on dryness of the skin, quantity of sebum, thickness of stratum corneum, or the like.

As a countermeasure therefor, it is conceivable to bring the working side electrode assembly and the non-working side electrode assembly into contact with the skin ormucosa after removing the sebum and the stratum corneum from the skin as much as possible.

However, about removing operation of the sebum and the stratum corneum, there occurs no trouble if the operation is conducted by a nurse or the like at a medical institution. However, if the iontophoresis device is used by a user by himself at his home, or the like, the above-mentioned removing operation is troublesome, thereby being not likely to conduct.

### Disclosure of the Invention

This invention has been made in view of the above-mentioned problem, and has an obj ect of the invention to provide an iontophoresis device which is constructed so that the iontophoresis device may be used after removing the sebum and the stratum corneum partially or fully at a region for administering the drug solution ion on the skin.

That is, the present invention provides an iontophoresis device including: a power source; and a working side electrode assembly and a non-working side electrode assembly connected to the power source, for administering a drug ion held in the working side electrode assembly to an organism from an organism contact surface at a tip of the working side electrode assembly through voltage applied from the power source, in which: the iontophoresis device further includes a release liner which is attached so as to cover the organism contact surface and is peelable by pulling the release liner in one direction along the organism contact surface; and on a front surface side of the release liner, in the organism contact surface, a base film, which is connected to the release liner at a vicinity of an end portion opposite to the pulling direction side and covers at least a front surface region of the organism contact surface, and a paste layer attached to the front surface of the base film are provided, the paste layer having an adhesive force which is peelable with respect to the skin by pulling the base film toward the pulling direction. Thus, the above-mentioned problem may be solved by mounting the device after removing even a little amount of the stratum corneum and the sebum of the skin prior to use of the device.

### Brief Description of the Drawings

[Fig. 1] An exploded perspective view illustrating an iontophoresis device according to Embodiment 1 of the present invention.
[Fig. 2] A sectional view of the iontophoresis device.
[Fig. 3] A sectional view schematically illustrating a process of exfoliating a base film in Embodiment 1.
[Fig. 4] A sectional view schematically illustrating an iontophoresis device according to Embodiment 2.
[Fig. 5] A sectional view schematically illustrating an iontophoresis device according to Embodiment 3.
[Fig. 6] A sectional view schematically illustrating an iontophoresis device according to Embodiment 4.
[Fig. 7] A sectional view schematically illustrating an exfoliating process for a paste layer in Embodiment 4.

### Best Mode for carrying out the Invention

Hereinafter, description is made of Embodiments 1 to 4 of the present invention.

### Embodiment 1

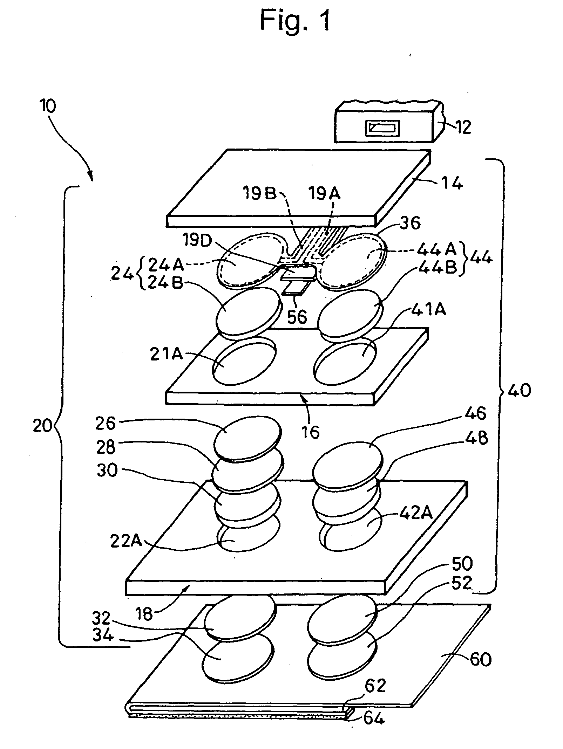

Next, an iontophoresis device 10 according to an Embodiment 1 of the present invention will be described in detail with reference to Figs. 1 to 2.

The iontophoresis device 10 is formed of a DC electric power source 12, a working electrode assembly 20 connected to one of an anode and cathode of the DC electric power source 12, and a non-working electrode assembly 40 connected to the other of the anode and cathode. The device administers a drug ion held by the working electrode assembly 20 to an organism with a voltage from the DC electric power source 12 through an organism contact surface 20A which being a tip surface of the working electrode assembly 20.

In the iontophoresis device 10, each of the working electrode assembly 20 and the non-working electrode assembly 40 is constructed by interposing constituent members such as a drug solution holding portion between a base end support 14 and an intermediate support 16, which are overlapped and are each formed of a resin sheet such as polyurethane foam, or by receiving the constituent members in through-holes formed in the intermediate support 16 and a tip support portion 18. The base end support 14 and the intermediate support 16 are formed into the same size, and the tip support 18 is formed so as to be larger than the supports 14 and 16.

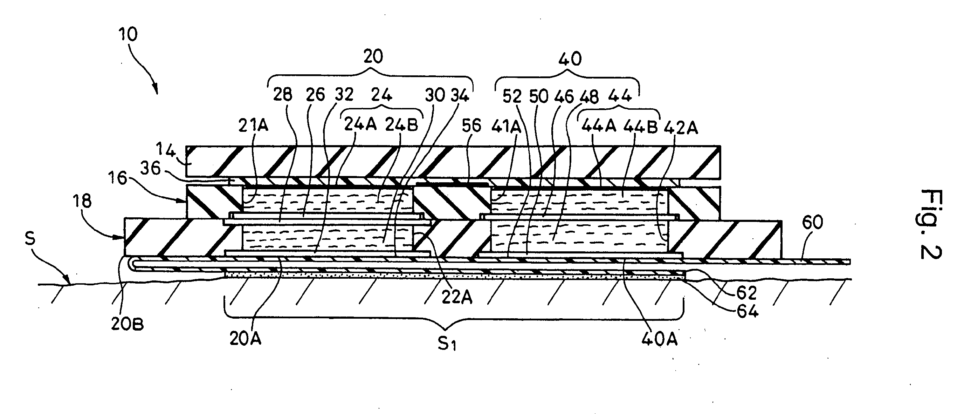

In Fig. 2, the base end support 14, the intermediate support 16, and the tip support 18 have a pressure-sensitive adhesive layers 14A, 16A, and 18A, respectively, at the lower surfaces thereof so as to be stuck to one another. At the same time, the pressure-sensitive adhesive layer 18A of the tip support 18 is stuck to the skin or mucosa.

Here, the intermediate support 16 is a single sheet-like member which forms a part of the working electrode assembly 20 and a part of the non-working electrode assembly 40.

Similarly, the tip support 18 is a single sheet-like member which forms a part of the working electrode assembly 20 and a part of the non-working electrode assembly 40.

The organism contact surface 20A of the working electrode assembly 20 and the organism contact surface 40A which being a tip surface of the non-working electrode assembly 40 are arranged flush with each other and with a gap. Further, there is provided a release liner 60 so as to cover the organism contact surfaces 20A and 40A being flush with each other.

The release liner 60 is peelable by pulling in one direction along the organism contact surfaces 20A and 40A, for example, in a right direction in Fig. 1.

Further, on a front surface side of the release liner 60, in the organism contact surfaces 20A, a base film 62, which is connected to the release liner 60 at a vicinity of an end portion 20B opposite to the pulling direction side and has a size capable of covering at least a front surface region of the organism contact surfaces 20A and 40A, and a paste layer 64 attached to the front surface of the base film 62 are provided.

The paste layer 64 has an adhesive force which is peelable with respect to the skin S by pulling the base film 62 toward the pulling direction, and is constructed so that a part of the stratum corneum and the sebum of the surface of the skin S may be simultaneously peeled off when the paste layer 64 is peeled off from the skin S.

The base film 62 is formed by bending the release liner 60 into a U shape at the vicinity of the end portion 20B and by extending the release liner. Here, the paste layer 64 is preferably made of a material, which adheres to the skin S but is hard to adhere to the organism contact surfaces 20A and 40A.

The structures of the working electrode assembly 20 and the non-working electrode assembly 40 are further described in detail.

The working electrode assembly 20 is formed by stacking a working electrode 24 connected to an anode and a cathode in a DC electric power source 12, which is the same polarity with a drug ion, a separator 26 arranged on a front surface of the working electrode 24, a second ion selective membrane 28 arranged on a front surface of the separator 26, selectively passing an ion opposite in sign to the drug ion, a drug solution holding portion 30 arranged on a front surface of the second ion selective membrane 28, for holding a drug which is different from the drug ion, a first ion selective membrane 32 arranged on a front surface of the drug solution holding portion 30, selectively passing an ion which is the same kind with the drug ion, and a working organism contact portion 34 which is formed by applying a viscous liquid containing a drug which is the same with the drug on a front surface of the first ion selective membrane 32 in the stated order. A front end surface of the working organism contact portion 34 is constituted as the working organism contact surface 20A which is brought into contact with the skin or mucosa.

The working electrode 24 is formed of: a working collector 24A connected to the DC electric power source 12 and formed of a carbon-printed electrode formed by printing on the front surface of a resin sheet 36; and a working polarizable electrode 24B placed while being electrically connected to the front surface of the working collector 24A.

It should be noted that the phrase "electrically connected" comprehends not only the case where the electrode is directly connected to the front surface but also the case where the electrode is connected to the front surface through an electric conductor such as a conductive adhesive (the same holds true for the following).

The intermediate support 16 is formed of a resin material having a thickness substantially equal to that of the working polarizable electrode 24B, and has a working intermediate through-hole 21A of substantially the same shape as that of an external shape in the plane shape of the working polarizable electrode 24B. The working polarizable electrode 24B is received in the working intermediate through-hole 21A.

In addition, the tip support 18 is formed of a resin material having a thickness substantially equal to that of the drug solution holding portion 30, and has a working tip through-hole 22A of substantially the same shape as that of an external shape in the plane shape of the working polarizable electrode 24B. The drug solution holding portion 30 is received in the working tip through-hole 22A.

The non-working electrode assembly 40 is formed by stacking, from the base end support 14 in the following order, a non-working electrode 44 connected to an anode or a cathode in a DC electric power source 12, which is an opposite polarity with a drug ion, a separator 46 arranged on a front surface of the non-working electrode 44, an electrolyte solution holding portion 48 for holding an electrolyte solution, a third ion selective membrane 50 selectively passing an ion opposite in sign to the drug ion, and a non-working organism contact portion 52 which is formed by applying a viscous liquid containing a same electrolyte solution which is the same with the electrolyte solution which is held in the electrolyte solution holding portion 48. A front end surface of the non-working organism contact portion 52 is constituted as the non-working organism contact surface 40A.

The non-working electrode 44 is formed of: a non-working collector 44A which is formed of a material containing carbon and printed in a film shape on the front surface of the resin sheet 36 so as to be apart from the working collector 24A of the working electrode 24; and a non-working polarizable electrode 44B provided so as to contact the non-working collector 44A.

The non-working polarizable electrode 44B has a thickness equal to that of the intermediate support 16, and is received in a non-working intermediate through-hole 41A formed in the intermediate support 16. In addition, the electrolyte solution holding portion 48 has a thickness equal to that of the tip support 18, and is received in a non-working tip through-hole 42A formed in the tip support 18.

In Embodiment 1, each of the through-holes 22A, 21A, 41A, and 42A is of a circular shape, and, furthermore, each of the working electrode 24, the separator 26, the second ion selective membrane 28, the drug solution holding portion 30, the first ion selective membrane 32, and the working organism contact portion 34 is of a circular membrane shape or a sheet shape.

Similarly, each of the non-working electrode 44, the separator 46, the electrolyte solution holding portion 48, the third ion selective membrane 50, and the non-working organism contact portion 52 is of a circular membrane shape or a sheet shape.

As illustrate in Fig. 1 by a dashed line, in the resin sheet 36, a working wire 19A and a non-working wire 19B which are formed of a material containing carbon and printed continuously in a film shape are connected to the working collector 24A in the working electrode 24 and the non-working collector 44A in the non-working electrode 44, respectively

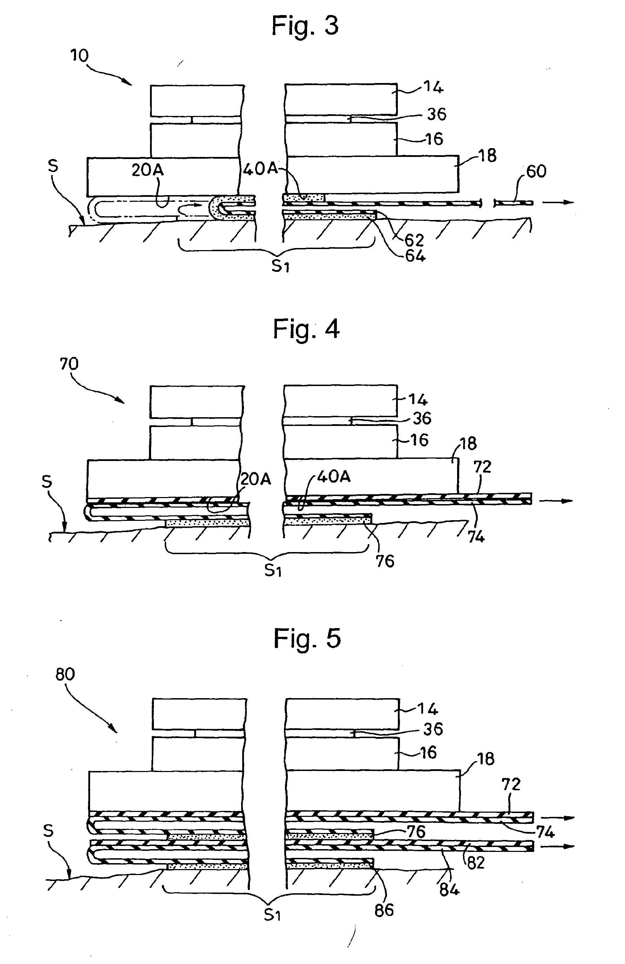

In Embodiment 1, as shown in each of Figs. 1 and 4, the respective circular members are overlapped in each of the working electrode assembly 20 and the non-working electrode assembly 40 in the thickness direction of each assembly, and are integrated to constitute the iontophoresis device 10.

Here, the intermediate support 16 is interposed between other members from above and below the support in a state where the working polarizable electrode 24B and the non-working polarizable electrode 44B are received in the working intermediate through-hole 21A and non-working intermediate through-hole 41A of the intermediate support 16, respectively. Similarly, the tip support 18 is interposed between other members from above and below the support in a state where the drug solution holding portion 30 and the electrolyte solution holding portion 48 are stored in the working tip through-hole 22A and non-working tip through-hole 42A of the tip support 18, respectively. Further, a member between the base end support 14 and the intermediate support 16 is positioned and fixed by being interposed between them, and a member between the intermediate support 16 and the tip support 18 is positioned and fixed by being interposed between them.

Each of outer diameters of the working collector 24A and the non-working collector 44A in the working electrode 24 and the non-working electrode 44 is slightly larger than each of diameters of the working intermediate through-hole 21A and non-working intermediate through-hole 41A. Outer peripheral portions thereof are arranged so as to be sandwiched between the base end support 14 and the intermediate support 16.

Reference numeral 56 of Fig. 1 and Fig. 2 represents an adhesive. The adhesive 56 is placed across an intermediate portion between the working and non-working collectors 24A and 44A in the insulating film 19D to bind the insulating film 19D and the intermediate support 16 so that the area between the insulating film 19D and the intermediate support 16 is partitioned into a working side and a non-working side.

In addition, reference numeral 58 of Fig. 1 represents a release liner attached to the front surface of the tip support 18 in a peelable fashion so as to cover the working organism contact portion 34 and the non-working organism contact portion 52.

Next, materials, components, and the like for the above respective constituent elements is described.

In Embodiment 1, the drug solution holding portion 30 is constructed by impregnating a polypropylene (PP) non-woven fabric with a viscous liquid containing the drug. In addition, the drug solution holding portion 30 is impregnated with a drug, the drug effect component of which dissociates into a positive or negative ion by, for example, dissolution in a solvent such as water (drug which dissociates into a drug ion (a precursor for the drug is also permitted)). A drug, the drug effect component of which dissociates into a positive ion is, for example, lidocaine hydrochloride as a narcotic or morphine hydrochloride as a narcotic. A drug, the drug effect component of which dissociates into a negative ion is, for example, ascorbic acid which being a vitamin agent.

In addition to the foregoing, a hormone, DNA, RNA, a protein, an amino acid, and minerals are also included in the category described above.

The separator 46 in the non-working electrode assembly 40 is obtained by impregnating a PP non-woven fabric with a viscous liquid containing the electrolyte solution (details about the electrolyte solution is described later). Further, the electrolyte solution holding portion 48 is obtained by impregnating a PP non-woven fabric with a viscous liquid containing the same electrolyte solution.

The electrolyte solution to be used in each of the separator 46 and the electrolyte solution holding portion 48 is mainly formed of an electrolyte, and electrolytes each of which is oxidized or reduced more readily than the electrolytic reaction of water (oxidation at an anode and reduction at a cathode) such as: pharmaceuticals such as ascorbic acid (vitamin C), and sodium ascorbate; and organic acids such as lactic acid, oxalic acid, malic acid, succinic acid, and fumaric acid and/or salts of the acids are each particularly preferably used as the electrolyte. As a result, the generation of an oxygen gas or hydrogen gas may be suppressed.
In addition, a fluctuation in pH of the electrolyte solution during energization may be suppressed by blending such a combination of multiple kinds of electrolytes as to provide a buffer electrolyte solution when being dissolved in a solvent.

Such viscous liquid containing the drug or electrolyte solution described above may be produced by, for example, mixing water (ion exchange water) with 2 mass% or more of a tacky material such as hydroxypropylcellulose (HPC) (for example, H-Type manufactured by NIPPON SODA CO. , LTD.) or a Metolose as a water-soluble polymer obtained by chemically treating a water-insoluble cellulose (for example, 90SH-10000SR manufactured by Shin-Etsu Chemical Co., Ltd.).

The working polarizable electrode 24B in the working electrode 24 and the non-working polarizable electrode 44B in the non-working electrode 44 are either constructed of as a main component a conductive base, which is formed of active carbon, preferably, a carbon fiber or carbon fiber paper. If an active carbon fiber only is used as the working and non-working polarizable electrodes 24B and 44B, it is preferred to form a layer by combining cloth and felt formed of the active carbon fiber. Beside, for the conductive base, for example, layers in which the active carbon is dispersed within a binder polymer may be laminated. As the active carbon, active carbon having a specific area of 10 m²/g or more may be used.

The working polarizable electrode 24B is impregnated with a viscous liquid containing a drug identical to the drug held by the drug solution holding portion 30. In addition, the non-working polarizable electrode 44B is impregnated with a viscous liquid containing an electrolyte solution identical to the electrolyte solution held by the separator 46.

Each of the working collector 24A in the working electrode 24 and the non-working collector 44A in the non-working electrode 44 is a printed electrode obtained by printing a polyethylene terephthalate (PET) material mixed with carbon and an adhesive.

It should be noted that, as a material for the working collector 24 and the non-working collector 44A, the material may be used as long as having conductivity, and a conductive metal such as gold, platinum, silver, copper, or zinc other than carbon may be used. Alternatively, a conductive material such as carbon or gold itself may be used as a collector without reliance on printing. In addition, as a material for the working wire 19A and the non-working wire 19B, the same may be applied.

The separator 26 is obtained by impregnating a PP non-woven fabric with a viscous liquid containing a drug identical to the drug held by the drug solution holding portion 30, and is interposed between the working polarizable electrode 24B and the second ion selective membrane 28 for preventing a physical contact between them.

The first ion selective membrane 32 is constructed by incorporating an ion-exchange resin into which an ion-exchange group is introduced so that an ion having the same sign with the drug ion is selectively caused to pass through the resin. Specifically, the first ion selective membrane 32 contains a cation exchange resin when the drug solution of the drug solution holding portion 30 dissociates into a cation, and contains an anion exchange resin when the drug solution dissociates into an anion.

The second ion selective membrane 28 is constructed by incorporating an ion-exchange resin into which an ion-exchange group is introduced so that an ion opposite in sign to the drug ion is selectively caused to pass through the resin. Specifically, the second ion selective membrane 28 contains an anion exchange resin when the drug solution of the drug solution holding portion 30 dissociates into a cation, and contains the cation exchange resin when the drug solution dissociates into the anion.

As in the case of the second ion selective membrane 28, the third ion selective membrane 50 is constructed by incorporating an ion exchange resin into which an ion exchange group is introduced so that an ion opposite in sign to the drug ion is selectively caused to pass through the resin. That is, when the drug solution of the drug solution holding portion 30 dissociates into a cation, the third ion selective membrane 50 contains an anion exchange resin, and when the drug solution dissociates into an anion, the membrane contains a cation exchange resin.

As the above cation exchange resin, there may be used, without any limitation, an ion exchange resin obtained by introducing a cation exchange group (exchange group the counter ion of which is a cation) such as a sulfonic group, a carboxylic group, or a phosphonic group into a polymer having a three-dimensional network structure such as a hydrocarbon-based resin such as a polystyrene resin or an acrylic acid-based resin, or a fluorine-based resin having a perfluorocarbon skeleton.

In addition, as the above anion exchange resin, there may be used, without any limitation, an ion exchange resin obtained by introducing a cation exchange group (exchange group, the counter ion of which is an anion) such as a primary, secondary, or tertiary amino group, a quaternary ammonium group, a pyridyl group, an imidazole group, a quaternary pyridium group, or a quaternary imidazolium group into the same polymer having a three-dimensional network structure as that in the case of the cation exchange resin.

The working organism contact portion 34 is constructed by applying a viscous liquid identical to a liquid, with which the drug solution holding portion 30 is impregnated to the front surface of the first ion selective membrane 32. In addition, the non-working organism contact portion 52 is constructed by applying a viscous liquid containing an electrolyte solution identical to that used in the electrolyte solution holding portion 48 to the front surface of the third ion selective membrane 50. It should be noted that each of those viscous liquids is desirably applied in an amount as small as 7 µL in order that the spread of each of the applied viscous liquids due to the attachment of the release liner 58 on each of the viscous liquids may be suppressed.

At the time of the assembly of the iontophoresis device 10, in a state shown in Fig. 1, the respective constituent members are placed like a laminate or stored in through-holes, and the base end support 14, the intermediate support 16, and the tip support 18 are sequentially overlapped on the release liner 58, and are fixed by being stuck to one another with the pressure-sensitive adhesive layers 16A and 14A. Thus, the assembly is completed.

A button cell or a thin cell disclosed in, for example, Japanese Patent Application Laid-Open No. Hei 11-067236, US Patent Publication No. 2004/0185667 A1, or US Patent No. 6,855,441 may be used as the DC electric power source 12, and the structure of the source is not limited to that in this embodiment.

When the above-mentioned iontophoresis device 10 is used, as illustrated in Fig. 2, the paste layer 64 of the base film 62 is adhered to a contact region S₁, which is a portion, in the skin S, with which the working organism contact surface 20A and the non-working organism contact surface 40A (hereinafter, referred to as organism contact surfaces 20A and 40A) are brought into contact.

Next, the release liner 60 is pulled toward a right direction in Fig. 2 in parallel with the organism contact surfaces 20A and 40A. In this case, for example, a user grasps the whole iontophoresis device 10, to thereby fix its position with respect to the skin S.

With this, the release liner 60 moves along the organism contact surfaces 20A and 40A, and the base film 62 is also pulled through the movement of the release liner 60, with the result that the paste layer 64 is, as illustrated in Fig. 3, peeled from the skin S sequentially from the left side. Apart of the stratum corneum or sebum of the skin S is adhered to the paste layer 64. This means that the stratum corneum or sebum is peeled from the contact region S₁ of the skin S.

The paste layer 64 peeled from the skin S moves along the organism contact surfaces 20A and 40A toward the right direction in the figure, and when ends (right ends in the figure) of the base film 62 and the paste layer 64 pass through the organism contact surfaces 20A and 40A, the peeling of the release liner 60 and the base film 62 are finished.

In this state, the iontophoresis device 10 is mounted so that the organism contact surfaces 20A and 40A are brought into contact with the skin S.

In the contact region S₁ with which those organism contact surfaces 20A and 40A are brought into contact, the part of the stratum corneum or sebum is peeled as described above, and hence impedance of the skin is small and the drug ion may be administered efficiently.

### Embodiment 2

Next, description is made of an iontophoresis device 70 according to Embodiment 2, which is illustrated in Fig. 4.

The iontophoresis device 70 is formed by incorporating the release liner 60, and a release liner 72 and a base film 74 each having a different structure from the base film 62. The other structure is the same with the structure of the iontophoresis device 10 according to Embodiment 1, and hence the detailed description of the same portion is omitted.

The release liner 72 according to Embodiment 2 is, as illustrated in Fig. 4, formed as a separate member from the base film 74, and is integrally formed on a front surface of the release liner 72. In addition, the release liner 72 is bent into a U-shape at the vicinity of an end portion 20B to be extended, and the paste layer 76 is formed on the extended portion.

In Embodiment 2, when the release liner 72 and the base film 74 which is integrally formed therewith are pulled toward a right direction in Fig. 4, while supporting the iontophoresis device 70, the paste layer 76 is sequentially peeled from the contact region S₁ of the skin S from a left end in Fig. 14. At this time, a part of the stratum corneum or sebum of the skin S may be peeled off.

In Embodiment 2, different from Embodiment 1, the peeled paste layer 76 is free from contacting with the organism contact surfaces 20A and 40A, and hence there are such advantages that the peeling of the release liner 72 from the organism contact surfaces 20A and 40A may be performed smoothly, and a selective range of the material for the paste layer 76 becomes wider.

### Embodiment 3

Next, description is made of an iontophoresis device 80 according to Embodiment 3, which is illustrated in Fig. 5.

The iontophoresis device 80 is constructed by adding a second release liner 82, a second base film 84, and a second paste layer 86 with respect to the iontophoresis device 70 according to Embodiment 2, which is illustrated in Fig. 4, and the other structures are the same with the iontophoresis device 70 according to Embodiment 2. Accordingly, the detailed description thereof is omitted.

The second release liner 82 in Embodiment 3 is provided to a lower side in Fig. 5 of the paste layer 76 which is attached to the base film 74. The second base film 84 is integrally formed with respect to the second release liner 82 at its front surface, and is also bent into a U-shape at the vicinity of the end portion 20B to be extended.

Specifically, the second release liner 82, the second base film 84, and the second paste layer 86 each having the same structure with the release liner 72, the base film 74, and the paste layer 76 in Embodiment 2 are newly provided in stack.

In the iontophoresis device 80 according to Embodiment 3, first, the second release liner 82 and the second base film 84 on the skin S side are pulled toward a right direction in Fig. 5, thereby peeling off the second paste layer 86 from the skin S, and the second release liner 82 is peeled off from the paste layer 76 above the second release liner 82.

Next, after adhering the paste layer 76 of the base film 74 onto the skin S, the release liner 72 and the base film 74 are pulled toward the right direction in Fig. 5, whereby the paste layer 76 peels off a part of the stratum corneum or sebum from the skin S as described above.

Like this, the part of the stratum corneum of sebum of the skin S are peeled off two times by the paste layers 86 and 76, and hence, compared to a case where the peeling is conducted one time, the impedance of the skin may be made smaller.

In Embodiment 3 described in Fig. 5, the part of the stratum corneum or the like is peeled off two times from the skin S by two layers of the paste layers 86 and 76. However, the present invention is not limited thereto, and a combination of the release liner, base film, and the paste layer may be provided in three layers.

### Embodiment 4

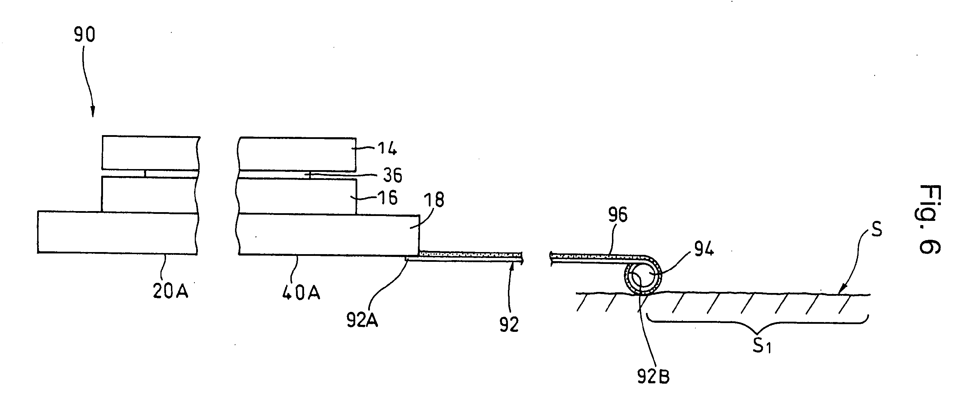

Next, description is made of an iontophoresis device 90 according to Embodiment 4 with reference to Fig. 6.

The iontophoresis device 90 has a substantially same shape and size which being a total of the organism contact surfaces 20A and 40A, and includes: a release liner 92 having one side end 92A which is attached at a right end portion of the non-working organism contact surfaces 40A in Fig. 6; and a winding member 94 attached to another side end 92B, which is opposite to the one side end 92A of the release liner 92, capable of winding the release liner 92, while rotating, from the another side end 92B toward the one side end 92A.

The release liner 92 has a paste layer 96 formed on a surface which is opposite to a surface which is brought into contact with the winding member 94, and constitutes a paste-attached liner. The paste layer 96 is constructed so as to have an adhesive force which is peelable with respect to the skin when the paste layer-attached release liner is rolled up.

In the iontophoresis device 90 according to Embodiment 4, as illustrated in Fig. 6, the paste layer 96 outside the winding member 94 is brought into contact with the contact region S₁ of the skin S, with which the organism contact surfaces 20A and 40A are to be brought into contact, from a left side in the figure, and then the winding member 94 is rotated, for example, toward the right direction.

If the winding member 94 is rotated toward the right direction in Fig. 6, the outside paste layer 96 is brought into contact with the contact region S₁, and further, when the paste layer 96 is taken up by the winding member 94, a part of the stratum corneum or sebum of the contact region S₁ is peeled off through the adhesive force of the paste layer 96.

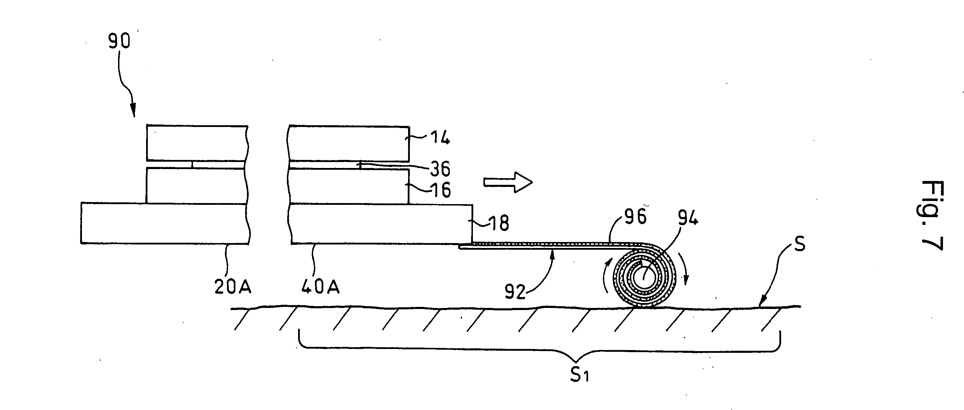

Through a way as described above, when the winding member 94 is caused to advance while taking up the release liner 92 along the contact region S₁, as illustrated in Fig. 7, the part of the stratum corneum or sebum in an entire area of the contact region S₁ is peeled off by the paste layer 96.

At a time of finish of the peeling, the organism contact surfaces 20A and 40A are positioned on an upper side of the contact region S₁. If electrification is conducted after removing the release liner 92 or as it is, without removing, and bringing the organism contact surfaces 20A and 40A into contact with the contact region S₁, the drug ion may efficiently be administered.

It should be noted that, in each of the iontophoresis devices 10, 70, 80, and 90 according to the above-mentioned embodiments, each of the paste layers 64, 76, 86, and 96 peels off a part of the stratum corneum, or the like from the contact region S₁ of the skin S in an area where the both the organism contact surfaces 20A and 40A are brought into contact with the paste layers 64, 76, 86, and 96, but the present invention is not limited to this. The part of the stratum corneum, or the like may only be peeled off from the skin S at least an area with which the working contact surface 20A of the working electrode assembly is brought into contact.

Further, the structure of the iontophoresis device main body is not limited to the structures according to Embodiments 1 to 4, but may be applied, in the working electrode assembly, to a case of including at least a working electrode and a drug solution holding portion.

Further, the structure of the iontophoresis device main body may be applied to a case where, without using the working organism contact portion 34 or non-working organism contact portion 52, the ion selective film constitutes the organism contact surface.

### Industrial Applicability

In this invention, the user itself may pull the base film, which is adhered with respect to the skin through the paste layer, by using the release liner covering the organism contact surface at least at the tip of the working electrode assembly of the working electrode assembly and the non-working electrode assembly, and may peel off the part of the stratum corneum or sebum from the drug solution ion administering area by causing the stratum corneum or sebum to be attached to the paste layer. As a result, there may be obtained the iontophoresis device which being easy for use.

## Claims

1. An iontophoresis device, comprising: a power source; and a working side electrode assembly and a non-working side electrode assembly connected to the power source, for administering a drug ion held in the working side electrode assembly to an organism from an organism contact surface at a tip of the working side electrode assembly through voltage applied from the power source, wherein:
the iontophoresis device further comprises a release liner which is attached so as to cover the organism contact surface and is peelable by pulling the release liner in one direction along the organism contact surface; and
on a front surface side of the release liner, in the organism contact surface, a base film, which is connected to the release liner at a vicinity of an end portion opposite to the pulling direction side and covers at least a front surface region of the organism contact surface, and a paste layer attached to the front surface of the base film are provided, the paste layer having an adhesive force which is peelable with respect to the skin by pulling the base film toward the pulling direction.

2. The iontophoresis device according to claim 1, wherein the base film is formed by bending the release liner into a U shape at the vicinity of the end portion and by extending the release liner.

3. The iontophoresis device according to claim 1, wherein the base film is provided on a front surface of the release liner integrally therewith, and is bent into a U shape at the vicinity of the end portion to be extended.

4. The iontophoresis device according to any one of claims 1 to 3, further comprising: a second release liner which is attached to a front surface of the paste layer so as to cover the paste layer, and is peelable by pulling the second release liner along the paste layer;
a second base film which is provided on a front surface side of the second release liner, in the paste layer, which is connected to the second release liner at least at a vicinity of an end portion opposite to the pulling direction and covers at least a front surface region of the organism contact surface, and a second paste layer attached to the front surface of the second base film, the second paste layer having an adhesive force which is peelable with respect to the skin by pulling the second base film toward the pulling direction.

5. The iontophoresis device according to claim 4, wherein the second base film is formed by bending the second release liner into a U shape at the vicinity of the end portion and by extending the release liner.

6. The iontophoresis device according to claim 4, wherein the second base film is provided on a front surface of the second release liner integrally therewith, and is bent into a U shape at the vicinity of the end portion to be extended.

7. An iontophoresis device, comprising: a power source; and a working side electrode assembly and a non-working side electrode assembly connected to the power source, for administering a drug ion held in the working side electrode assembly to an organism from an organism contact surface at a tip of the working side electrode assembly through voltage applied from the power source, wherein:
the iontophoresis device further comprises:
a paste layer-attached release liner having a substantially same shape and size with the organism contact surface, one side end of the release liner being attached to one end portion of the organism contact surface; and
a winding member attached to another side end which is opposite to the one side end of the paste layer-attached release liner, capable of winding the paste layer-attached release liner from the other side end toward the one side end on one side, and
the paste layers are provided on a surface that is brought into contact with the winding member in the paste layer-attached release liner and a surface opposite thereto, the paste layers having an adhesive force which is peelable with respect to the skin when the paste layer-attached release liner is rolled up.

8. The iontophoresis device according to any one of claims 1 to 7, wherein the organism contact surface comprises an organism contact surface at a tip of the non-working side electrode assembly.
